(19) Europäisches Patentamt
European Patent Office
Office européen des brevets

(11) EP 4 083 004 A1

(12) EUROPEAN PATENT APPLICATION
published in accordance with Art. 153(4) EPC

(43) Date of publication:
02.11.2022 Bulletin 2022/44

(21) Application number: 20906588.7

(22) Date of filing: 23.12.2020

(51) International Patent Classification (IPC):
$C07C\ 29/80^{(2006.01)}$  $C07C\ 31/20^{(2006.01)}$
$A61Q\ 19/00^{(2006.01)}$  $C07C\ 37/74^{(2006.01)}$
$A61K\ 8/34^{(2006.01)}$

(52) Cooperative Patent Classification (CPC):
A61K 8/34; A61Q 19/00; C07C 29/80; C07C 31/20;
C07C 37/74

(86) International application number:
PCT/JP2020/048220

(87) International publication number:
WO 2021/132360 (01.07.2021 Gazette 2021/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.12.2019 JP 2019239974
28.12.2019 JP 2019239975
28.12.2019 JP 2019239976
28.12.2019 JP 2019239977
28.12.2019 JP 2019239978
28.12.2019 JP 2019239979
20.01.2020 JP 2020006660
06.02.2020 JP 2020018910

(71) Applicant: Daicel Corporation
Osaka 530-0011 (JP)

(72) Inventors:
• SHIMIZU, Masahiko
Tokyo 108-8230 (JP)
• MIZUTANI, Yoshihisa
Tokyo 108-8230 (JP)

(74) Representative: Grünecker Patent- und
Rechtsanwälte
PartG mbB
Leopoldstraße 4
80802 München (DE)

(54) **METHOD FOR PRODUCING 1,3-BUTYLENE GLYCOL, AND 1,3-BUTYLENE GLYCOL PRODUCT**

(57)     Provided is a method capable of manufacturing 1,3-butylene glycol having a high potassium permanganate test value. A method for manufacturing 1,3-butylene glycol, which is a method for obtaining purified 1,3-butylene glycol from a crude reaction liquid containing 1,3-butylene glycol, the method including: a dehydration step of removing water by distillation; a high boiling substance removal step of removing a high boiling point component by distillation; and a product distillation step of obtaining purified 1,3-butylene glycol, wherein in the product distillation step, a product column is used in which a liquid feed having a 1,3-butylene glycol concentration of 97% or higher, an acetaldehyde content of 500 ppm or lower, and a crotonaldehyde content of 200 ppm or lower is distilled under a condition of a reflux ratio of higher than 0.1, and a liquid concentrated with acetaldehyde and crotonaldehyde is distilled off from above a feed plate, and 1,3-butylene glycol is extracted from below the feed plate.

FIG. 1

EP 4 083 004 A1

**Description**

Technical Field

The present disclosure relates to a method for manufacturing 1,3-butylene glycol, and a 1,3-butylene glycol product. The present patent application claims priority from the Japanese Patent Application No. 2019-239974, Japanese Patent Application No. 2019-239975, Japanese Patent Application No. 2019-239976, Japanese Patent Application No. 2019-239977, Japanese Patent Application No. 2019-239978 and Japanese Patent Application No. 2019-239979, all filed in Japan on December 28, 2019, the Japanese Patent Application No. 2020-006660 filed in Japan on January 20, 2020, and the Japanese Patent Application No. 2020-018910 filed in Japan on February 6, 2020, the entire contents of which are incorporated herein by reference.

Background Art

1,3-Butylene glycol is a colorless, transparent, and odorless liquid and has properties, such as low volatility, low toxicity, and high hygroscopicity, and has excellent chemical stability. 1,3-butylene glycol has a wide range of applications, including raw materials for various synthetic resins and surfactants, as well as materials for cosmetics, hygroscopic agents, high boiling point solvents, and antifreezes, etc. Particularly in recent years, 1,3-butylene glycol has been attracting attention for having excellent properties as a moisturizer, and demand is growing in the cosmetic industry.

One of the product standards for 1,3-butylene glycol includes a potassium permanganate test value (abbreviation: PMT). Patent Document 1 discloses 1,3-butylene glycol having a potassium permanganate fading time of 5 minutes or longer after 3 months of manufacture.

Citation List

Patent Document

Patent Document 1: JP 2001-213825 A

Summary of Invention

Technical Problem

However, in the known method described above, the potassium permanganate test value of the obtained 1,3-butylene glycol product is not always fully satisfactory. Also, the substance that caused to reduce the potassium permanganate test value of 1,3-butylene glycol products has not been identified.

An object of the present disclosure thus is to provide a method capable of manufacturing 1,3-butylene glycol having a high potassium permanganate test value.

Another object of the present disclosure is to provide a 1,3-butylene glycol product having a high potassium permanganate test value.

Still another object of the present disclosure is to provide a moisturizer and a cosmetic product that have excellent moisturizing performance and can maintain high quality for a long period of time.

Solution to Problem

As a result of diligent research to achieve the above-described purpose, the inventors according to the present disclosure have found that, when a liquid feed having a concentration of 1,3-butylene glycol and an acetaldehyde content within predetermined ranges is fed to a product column and distilled at a predetermined reflux ratio, a potassium permanganate test value of a 1,3-butylene glycol product can be greatly improved; and also that, when a distillate from the product column is recycled to a step prior to a product distillation step, a potassium permanganate test value of the 1,3-butylene glycol product can be further improved; and a recovery rate of 1,3-butylene glycol can be maintained and improved even when such a method is performed. The present disclosure has been completed based on these findings.

Specifically, the present disclosure provides a method for manufacturing 1,3-butylene glycol, which is a method for obtaining purified 1,3-butylene glycol from a crude reaction liquid containing 1,3-butylene glycol, the method including: a dehydration step of removing water by distillation; a high boiling substance removal step of removing a high boiling point component by distillation; and a product distillation step of obtaining purified 1,3-butylene glycol, wherein in the product distillation step, a product column is used in which a liquid feed having a 1,3-butylene glycol concentration of 97% or higher, an acetaldehyde content of 500 ppm or lower, and a crotonaldehyde content of 200 ppm or lower is distilled under a condition of a reflux ratio of higher than 0.1, and a liquid concentrated with acetaldehyde and crotonaldehyde is distilled off from above a feed plate, and 1,3-butylene glycol is extracted from below the feed plate.

The crude reaction liquid containing 1,3-butylene glycol may be a crude reaction liquid obtained by hydrogen reduction of an acetaldol.

The manufacturing method may further include an alkaline treatment step of treating a process stream containing 1,3-butylene glycol with a base.

Furthermore, the manufacturing method may further include a desalting step of removing a salt in a process stream containing 1,3-butylene glycol.

Furthermore, the manufacturing method may further include a dealcoholization step of removing a low boiling substance containing alcohols in a process stream containing 1,3-butylene glycol.

A reflux ratio of the product column may be 0.2 or higher.

The content of acetaldehyde in the liquid feed into the product column may be 205 ppm or lower.

The content of crotonaldehyde in the liquid feed into the product column may be 110 ppm or lower.

A distillation rate in the product column may be lower than 20 wt.%.

The number of theoretical plates of the product columns is, for example, from 1 to 100 plates.

At least a portion of a distillate from the product column may be recycled to a step prior to the product distillation step, namely the dehydration step, a dealcoholization step, a low boiling substance removal step, or another step prior to these steps.

The crude reaction liquid containing 1,3-butylene glycol may be a crude reaction liquid obtained by hydrogen reduction of acetaldols, and at least a portion of the distillate from the product column may be recycled to the hydrogen reduction of acetaldols or a step upstream of the hydrogen reduction.

An amount of the distillate from the product column being recycled to a step prior to the product distillation step may be lower than 30 wt.% with respect to a charged amount into the product column within a range not higher than a distilled amount in the product column.

The amount of the distillate from the product column being recycled to a step prior to the product distillation step may be 0.01 wt.% or higher with respect to a charged amount into the product column within a range not higher than the distilled amount in the product column.

The present disclosure also provides a 1,3-butylene glycol product having a potassium permanganate test value of longer than 10 minutes.

In the 1,3-butylene glycol product, a peak area ratio of 1,3-butylene glycol is preferably higher than 98.5%, according to a gas chromatographic analysis performed under conditions set forth below. Conditions for the gas chromatographic analysis are as follows:

Analytical Column: a column with dimethylpolysiloxane as a stationary phase, having a length of 30 m, an inner diameter of 0.25 mm, and a film thickness of 1.0 $\mu$m

Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes

Sample Introduction Temperature: 250°C

Carrier Gas: helium

Column Gas Flow Rate: 1 mL/min

Detector and Detection Temperature: a flame ionization detector (FID), 280°C.

The 1,3-butylene glycol product preferably has an acetaldehyde content of 2 ppm or lower.

The 1,3-butylene glycol product preferably has a crotonaldehyde content of 1.2 ppm or lower.

The present disclosure further relates to a moisturizer containing the 1,3-butylene glycol product.

Furthermore, the present disclosure further provides a cosmetic product containing the moisturizer.

In the present disclosure, "1,3-butylene glycol product" means a composition in which 1,3-butylene glycol occupies a majority of the components (e.g., a 1,3-butylene glycol content is 95 wt.% or higher, preferably 98 wt.% or higher).

Advantageous Effects of Invention

The manufacturing method of the present disclosure is capable of manufacturing 1,3-butylene glycol having a high potassium permanganate test value.

The 1,3-butylene glycol product of the present disclosure has a high potassium permanganate test value. Therefore, it is suitably used as a moisturizer, and in an application as a raw material for cosmetics.

Further, the moisturizers and cosmetic product of the present disclosure are excellent in moisturizing performance and have an extremely low content of reducing substances, and thus can maintain high quality for a long period of time.

Brief Description of Drawings

FIG. 1 is a flowchart of a manufacturing method (purification method) for a 1,3-butylene glycol product of the present disclosure.
FIG. 2 is a chromatogram showing a gas chromatographic analysis for a 1,3-butylene glycol product in Example 13.
FIG. 3 is a chromatogram showing a gas chromatographic analysis for a 1,3-butylene glycol product in Comparative Example 2.

Description of Embodiments

Method for manufacturing 1,3-butylene glycol

The method for manufacturing 1,3-butylene glycol of the present disclosure is a method for manufacturing 1,3-butylene glycol, in which purified 1,3-butylene glycol is obtained from a crude reaction liquid containing 1,3-butylene glycol (1,3 BG) (hereinafter sometimes referred to as "crude 1,3-butylene glycol"), the method including: a dehydration step of removing water by distillation; a high boiling substance removal step of removing a high boiling point component by distillation; and a product distillation step of obtaining purified 1,3-butylene glycol. In the product distillation step, a product column is used in which a liquid feed having a 1,3-butylene glycol concentration of 97% or higher, an acetaldehyde content of 500 ppm or lower, and a crotonaldehyde content of 200 ppm or lower is distilled under a condition of a reflux ratio of higher than 0.1, and a liquid concentrated with acetaldehyde and crotonaldehyde is distilled off from above a feed plate, and 1,3-butylene glycol is extracted from below the feed plate. The 1,3-butylene glycol has a high potassium permanganate test value, and thus can be made into a 1,3-butylene glycol product.

Crude 1,3-butylene glycol

Examples of the crude 1,3-butylene glycol include: (1) a crude reaction liquid obtained by reduction (hydrogenation) of acetaldols; (2) a crude reaction liquid obtained by hydrolyzation of 1,3-butylene oxide; (3) a crude reaction liquid obtained by selective hydrocracking of erythritol; (4) a crude reaction liquid obtained by selective water addition to butadiene; (5) a crude reaction liquid obtained by hydrogen addition to n-butanal-3-one; (6) a crude reaction liquid obtained by hydrogen addition to 1-butanol-3-one; (7) a crude reaction liquid obtained by hydrogen addition to 3-hydroxy-1-butanoic acid; (8) a crude reaction liquid obtained by hydrogen addition to $\beta$-butyrolactone; and (9) a crude reaction liquid obtained by hydrogen addition to diketene. In the present disclosure, the crude 1,3-butylene glycol may be one, or a mixture of two or more, of the above (1) to (9). The crude 1,3-butylene glycol is preferably (1) the crude reaction liquid obtained by reduction (in particular, liquid phase reduction) of acetaldols.

Hereinafter, a case where the crude reaction liquid obtained by reduction (hydrogenation) of acetaldols is used as the crude 1,3-butylene glycol will be mainly described. Note that the reduction (hydrogenation) of acetaldols is sometimes referred to as "hydrogenation step".

The acetaldols used as a raw material in the hydrogenation step are not particularly limited, as long as they are a compound that yields 1,3-butylene glycol by hydrogen reduction. Examples of the raw material acetaldols include acetaldol; its cyclic dimer paraldol; aldoxane as a cyclic trimer of acetaldehyde; and mixtures of these.

The method of manufacturing the acetaldols (e.g., acetaldol or paraldol) is not particularly limited, but the acetaldol may be, for example, those obtained by an aldol condensation reaction of acetaldehyde in the presence of a basic catalyst or those obtained by pyrolysis or the like of aldoxane. Note that the manufacture of the acetaldols is sometimes referred to as "acetaldol manufacture" or "acetaldehyde polymerization".

A crude reaction liquid obtained by the reaction described above and containing acetaldols may be neutralized with an acid and used in the manufacturing of 1,3-butylene glycol. Such a crude reaction liquid may contain, in addition to acetaldols, acetaldehyde, crotonaldehyde, another aldehyde component; a low boiling point substance; a high boiling point substance, such as an aldehyde dimer or trimer; water; a salt; and the like. In the present specification, a compound having a lower boiling point than 1,3-butylene glycol may be referred to as a "low boiling point substance" or "low boiling substance", and a compound having a higher boiling point than 1,3-butylene glycol may be referred to as a "high boiling point substance" or "high boiling substance".

The crude reaction liquid containing acetaldols may be subjected to a pretreatment, such as dealcoholization distillation, dehydration distillation, desalting, alkaline treatment and dealkalization treatment, or impurity removal, as necessary, and a product obtained by removing by-products, such as unreacted acetaldehyde and crotonaldehyde, may be used. Examples of the pretreatment method include distillation, adsorption, ion exchange, conversion to a high boiling point substance by heating, and decomposition. For the distillation, a distillation method of various types, such as reduced pressure, normal pressure, increased pressure, azeotropic, extraction, or reaction, can be used. In particular, it is preferred that the crude reaction liquid containing acetaldols is subjected to simple evaporation, distillation, or hydrogen addition to remove aldehydes such as acetaldehyde and crotonaldehyde, followed by the hydrogenation step.

The content of the acetaldols in the raw material for hydrogenation is not particularly limited but is, for example, preferably 30 wt.% or higher (e.g., from 30 to 99 wt.%), more preferably 40 wt.% or higher (for example, from 40 to 98 wt.%), 50 wt.% or higher (for example, from 50 to 97 wt.%) or 60 wt.% or higher (for example, from 60 to 95 wt.%), and even more preferably from 65 to 90 wt.%, particularly preferably from 70 to 90 wt.%, and most preferably from 75 to 90 wt.%. With the content of the acetaldol within the above ranges, impurities contained in the crude reaction liquid containing 1,3-butylene glycol (crude 1,3-butylene glycol) tend to be reduced.

The raw material for hydrogenation may or may not contain water but preferably contains water from the viewpoint of the purity of 1,3-butylene glycol product. The water content in the raw material for hydrogenation is not particularly limited but is, for example, preferably 2 wt.% or higher, more preferably 5 wt.% or higher, even more preferably 10 wt.% or higher, and particularly preferably 15 wt.% or higher. The upper limit may be, for example, 90 wt.%, 80 wt.%, 70 wt.%, 60 wt.%, 50 wt.%, 40 wt.%, 30 wt.% or 20 wt.%. With the water content within the above ranges, the acetal of 1,3-butylene glycol and acetaldol contained in the resulting crude 1,3-butylene glycol is decreased, and thus this tends to increase the purity of the 1,3-butylene glycol product finally obtained. This is because the raw material for hydrogenation contains water to a certain extent, and the acetal is hydrolyzed into 1,3-butylene glycol accordingly as well as coexisting acetaldol is reduced into 1,3-butylene glycol.

Examples of the hydrogenation catalyst include Raney nickel. The hydrogenation catalyst can be used in a suspended state, or can also be added to a reaction vessel and used. The amount of the hydrogenation catalyst to be used is not particularly limited but is, for example, preferably from 1 to 30 parts by weight, more preferably from 4 to 25 parts by weight, even more preferably from 8 to 20 parts by weight, and particularly preferably from 12 to 18 parts by weight relative to 100 parts by weight of the raw material for hydrogenation. The amount of hydrogen to be used in the reduction reaction is not particularly limited but is, for example, preferably from 0.5 to 40 parts by weight, more preferably from 1 to 30 parts by weight, even more preferably from 4 to 20 parts by weight, and particularly preferably from 8 to 12 parts by weight relative to 100 parts by weight of the raw material for hydrogenation. A pressure (total pressure; gauge pressure) in a reaction system in the reduction reaction is not particularly limited, but is, for example, preferably from 9 to 70 MPa and more preferably from 10 to 40 MPa. A hydrogen pressure (partial pressure of hydrogen) in the reaction system is not particularly limited, but is, for example, from 7 to 60 MPa, and preferably from 10 to 30 MPa. The reaction temperature in the reduction reaction is not particularly limited but is, for example, preferably from 40 to 150°C, preferably from 50 to 140°C, and more preferably from 60 to 130°C. The reaction time (residence time) in the reduction reaction is not particularly limited but is, for example, from 10 to 500 minutes, preferably from 20 to 400 minutes, more preferably from 30 to 300 minutes, even more preferably from 50 to 280 minutes, and particularly preferably from 80 to 250 minutes. The present reaction can be carried out in any of a batch, semi-batch, or continuous manner.

For example, the thus-obtained crude 1,3-butylene glycol contains acetaldehyde (AD); butylaldehyde; crotonaldehyde (CR); acetone; a low boiling point substance (low boiling point compound) having an unsaturated bond, such as methyl vinyl ketone; a condensate of these; a condensate of 1,3-butylene glycol and the above low boiling point substance (e.g., an acetal of 1,3-butylene glycol and acetaldol); an alcohol such as ethanol, isopropyl alcohol, or butanol; water (for example, solvent), a salt produced by neutralization or the like, a catalyst (when used in suspension) or the like. By removing these impurities in the purification step, a 1,3-butylene glycol product (purified 1,3-butylene glycol) can be obtained.

Purification of crude 1,3-butylene glycol

The manufacturing method according to the present disclosure includes, at least, a dehydration step of removing water by distillation, a high boiling substance removal step of removing a high boiling point component by distillation (high boiling point substance removal distillation), and a product distillation step of obtaining purified 1,3-butylene glycol. The dehydration step and the high boiling substance removal step are both performed before the product distillation step, but the order of the dehydration step and the high boiling substance removal step does not matter. The manufacturing method according to the present disclosure may include, in addition to these steps, a desalting step, an alkaline reaction step (alkaline treatment), and a dealkalization step. Additionally, prior to the dehydration step, a catalyst separation step, a neutralization step by alkali, a dealcoholization step (low boiling substance removal step), and the like can be provided. These steps may be performed in the order described above, but the order of these steps may be changed as appropriate except that the dealkalization step is provided after the alkaline reaction step. For example, the dealcoholization step (low boiling substance removal step), the desalting step, the alkaline reaction step, and the dealkalization step can be performed in an appropriate order, but are usually performed after the hydrogenation step. Note that, among the above-described steps, the catalyst separation step, the neutralization step by alkali, the dealcoholization step (low boiling substance removal), the desalting step, the alkaline reaction step, and the dealkalization step may be performed as necessary, and do not necessarily have to be performed.

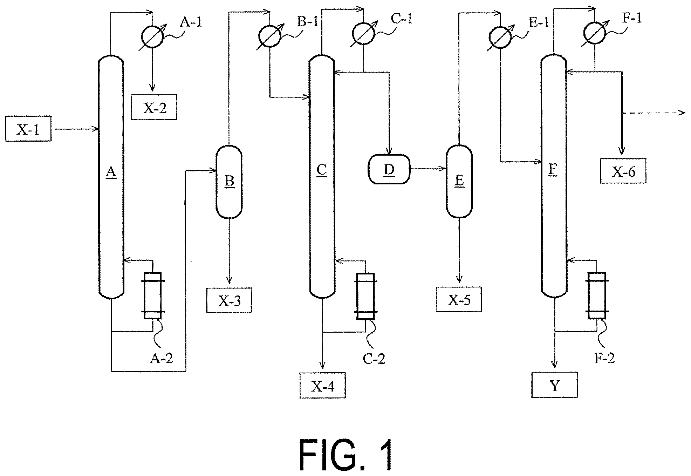

FIG. 1 is a flow sheet of an apparatus illustrating an example of an embodiment of a method for manufacturing 1,3-butylene glycol according to the present disclosure. A is a dehydration column and is related to the dehydration step. B is a desalting column and is related to the desalting step. C is a distillation column for removing a high boiling substance (high boiling substance removal column) and is related to the high boiling substance removal distillation step (high boiling substance removal). D is an alkaline reactor and is related to the alkaline reaction step. E is a dealkalization column and is related to the dealkalization step. F is a product distillation column (product column) and is related to the product distillation step. A-1, B-1, C-1, E-1, and F-1 are condensers. A-2, C-2, and F-2 are reboilers. Hereinafter, an example of an embodiment of the method for manufacturing 1,3-butylene glycol according to the present disclosure will be described using the present flow sheet.

Crude 1,3-butylene glycol (corresponding to "X-1") obtained by hydrogen reduction of a raw material for hydrogenation is fed to the dehydration column A. In the dehydration column A, water is distilled off from the top of the column by distillation, and from the bottom of the column is obtained a crude 1,3-butylene glycol stream containing 1,3-butylene glycol. Note that the crude 1,3-butylene glycol (corresponding to "X-1") may be fed to the dehydration column A after undergoing the dealcoholization (distillation by a dealcoholization column) for removing an alcohol such as ethanol and a low boiling point substance.

The crude 1,3-butylene glycol stream is fed to the desalting column B. In the desalting column B, the crude 1,3-butylene glycol stream after the desalting is obtained from the top of the column, and a salt, a high boiling point substance, or the like is discharged from the bottom of the column. The bottom rate (%) of the desalting column B [(desalting column bottom amount (part)/desalting column charged amount (part) × 100] is, for example, from 0.1 to 40 wt.%, preferably from 1 to 35 wt.%, more preferably from 2 to 30 wt.%, even more preferably from 3 to 25 wt.%, and particularly preferably from 5 to 20 wt.%, and may be from 7 to 15 wt.%. At least a portion of the bottom in the desalting column may be recycled to the step prior to the desalting.

The crude 1,3-butylene glycol stream after the desalting described above is fed to the high boiling substance removal column C. In the high boiling substance removal column C, the high boiling point component (preferably, high boiling point substance) is discharged from below the feed plate (preferably, from the bottom of the column). Meanwhile, the crude 1,3-butylene glycol stream after high boiling point substance removal (higher-purity 1,3-butylene glycol) is obtained from above the feed plate.

The high boiling substance removal column C can be, for example, a perforated-plate column, a bubble column, or the like, but is more preferably a packed column with a low pressure loss, filled with Sulzer Packing, Melapack (trade names of Sumitomo Heavy Industries, Ltd.). This is because 1,3-butylene glycol and trace impurities would be thermally decomposed at a high temperature (e.g., 150°C or higher) and produce a low boiling point substance, which is a coloring component, and thus the distillation temperature is to be lowered. In addition, this is also because a long thermal history (residence time) for 1,3-butylene glycol would also have a similar effect. Thus, the reboiler employed is preferably one with a short residence time of the process side fluid, for example, a thin-film evaporator, such as a natural downward flow thin-film evaporator or a forced-stirring thin-film evaporator.

The number of theoretical plates of the high boiling substance removal column C is, for example, from 1 to 100 plates, preferably from 2 to 90 plates, more preferably from 3 to 80 plates, more preferably from 4 to 70 plates, from 5 to 60 plates, from 8 to 50 plates, or from 10 to 40 plates, and particularly preferably from 15 to 30 plates. A feed position for the liquid feed is, for example, from 10 to 90%, preferably from 20 to 80%, and more preferably from 30 to 70 plates, and even more preferably from 40 to 60% of a height of the column facing downward from the top of the high boiling substance removal column. In the distillation in the high boiling substance removal column C, a pressure (absolute pressure) at the top of the column is, for example, from 0.01 to 50 kPa, preferably from 0.1 to 30 kPa, more preferably from 0.3 to 20 kPa, and even more preferably from 0.5 to 10 kPa.

The reflux ratio in the high boiling substance removal column C [high boiling substance removal column reflux amount/high boiling substance removal column distilled amount (discharge amount to outside of distillation column)] is, for example, 0.015 or higher, preferably 0.02 or higher, 0.03 or higher, 0.05 or higher, 0.07 or higher, 0.1 or higher, 0.2 or higher, 0.3 or higher, 0.4 or higher, 0. 5 or higher, 0.6 or higher, 0.7 or higher, 0.8 or higher, 0.9 or higher, 1 or higher, 2 or higher, 3 or higher, 4 or higher, 5 or higher, 6 or higher, 7 or higher, 8 or higher, 9 or higher, 10 or higher, 15 or higher, or 20 or higher, and more preferably 30 or higher. An upper limit of the reflux ratio is, for example, 100, preferably 50 from the point of energy cost.

The crude 1,3-butylene glycol stream taken out from above the feed plate of the high boiling substance removal column C is fed to the alkaline reactor (e.g., a flow-through tubular reactor) D and is treated with a base (treated with alkali). The base treatment can decompose by-products contained in the crude 1,3-butylene glycol. The base is added to the alkaline reactor D or its upstream piping or the like. The base is added in an amount of, for example, from 0.05 to 10 wt.%, preferably from 0.1 to 1.0 wt.% relative to the crude 1,3-butylene glycol stream subjected to the alkaline treatment. With the added amount of the base exceeding 10 wt.%, the base would precipitate in the distillation column, piping, or the like, and this may cause blockage. In addition, the decomposition reaction of a high boiling point compound would occur, and by-products may be produced on the contrary. With the added amount of the base of lower than 0.05 wt.%, the effect of decomposing byproducts is reduced.

The base added in the alkaline reactor D or its upstream piping is not particularly limited but is, for example, preferably an alkali metal compound. Examples of the alkali metal compound include sodium hydroxide, potassium hydroxide, sodium (bi)carbonate, and potassium (bi)carbonate. A basic ion exchange resin can also be used as the base. The base is preferably sodium hydroxide or potassium hydroxide from the perspective of reducing the byproducts contained in the 1,3-butylene glycol product obtained finally. The base may be added as is in the solid form but is preferably added in an aqueous solution to facilitate operation and contact with a solution to be treated. One of the bases described above may be used alone, or two or more may be used simultaneously.

The reaction temperature in the alkaline reactor D is not particularly limited but is, for example, preferably from 90 to 140°C and more preferably from 110 to 130°C. The reaction at a reaction temperature lower than 90°C would require long reaction residence time and thus require a reactor with a large volume and make the process uneconomical. The reaction at a reaction temperature exceeding 140°C would increase coloration in the 1,3-butylene glycol product obtained finally. The reaction residence time is, for example, preferably from 5 to 120 minutes and more preferably from 10 to 30 minutes. A reaction residence time shorter than 5 minutes may cause an insufficient reaction and deteriorate the quality of the 1,3-butylene glycol product obtained finally. A reaction residence time exceeding 120 minutes would require a large reactor and increase the cost of equipment, and thus would be disadvantageous from the economic point of view.

After exiting the alkaline reactor D, the crude reaction liquid stream is fed to the dealkalization column (e.g., thin film evaporator) E according to need, and the base and the like are removed from the bottom of the column by evaporation. Meanwhile, from the top of the dealkalization column E is obtained a crude 1,3-butylene glycol stream after the removal of a base. The evaporator used for the dealkalization column E is suitably a natural downward flow thin-film evaporator or a forced-stirring thin-film evaporator with a short residence time for the purpose of reducing the thermal history to the process fluid. A demister may be installed in a space above the charging position of the dealkalization column (e.g., thin film evaporator) E, and droplets of a base or the like may be removed. This makes it possible to prevent the base and the high boiling point substance from being mixed into the 1,3-butylene glycol product.

Evaporation is performed in the evaporator used for the dealkalization column E, for example, under a reduced pressure at the top of the column of 20 kPa or lower (absolute pressure), preferably from 0.5 to 10 kPa (absolute pressure). The temperature of the evaporator is, for example, preferably from 90 to 120°C. The crude 1,3-butylene glycol stream containing a low boiling point substance distilled off from the top of the column is fed to the product distillation column (product column) F.

Note that the alkaline reactor D and the dealkalization column E may be installed between the desalting column B and the high boiling substance removal column C, or between the dehydration column A and the desalting column B (in this case, the desalting column may also serve as a dealkalization column), or before the dehydration column A. In addition, without providing the alkaline reactor D or the dealkalization column E, the alkaline treatment can be performed by adding the base into a high boiling substance removal column charging line or into a dehydration column charging line, or adding the base to the reaction solution after the hydrogenation [and then charging the dealcoholization column (low boiling substance removal column) with the solution].

In the manufacturing method according to the present disclosure, in the product column F for use in the product distillation step, a liquid feed having a 1,3-butylene glycol concentration of 97% or higher, an acetaldehyde content of 500 ppm or lower, and a crotonaldehyde content of 200 ppm or lower is distilled under a condition of a reflux ratio of higher than 0.1, and a liquid concentrated with acetaldehyde and crotonaldehyde is distilled off from above a feed plate (corresponding to "X-6" in FIG. 1), and 1,3-butylene glycol is extracted from below the feed plate (corresponding to "Y" in FIG. 1). The 1,3-butylene glycol has a high potassium permanganate test value, and thus can be made into a 1,3-butylene glycol product as it is.

Examples of the product column F include perforated-plate columns and bubble columns, but more preferred is a packed column with a low pressure loss, filled with Sulzer Packing, Melapack (trade names of Sumitomo Heavy Industries, Ltd.), or the like. This is because 1,3-butylene glycol would be thermally decomposed at a high temperature (e.g., 150°C or higher) and produce a low boiling substance, which is a coloring component, and thus the distillation temperature is to be lowered. In addition, this is also because a long thermal history (residence time) for 1,3-butylene glycol would have a similar effect. Thus, the reboiler employed is preferably one with a short residence time of the process side fluid, for example, a thin-film evaporator, such as a natural downward flow thin-film evaporator or a forced-stirring thin-film evaporator.

The number of theoretical plates of the product column F is, for example, from 1 to 100 plates, preferably from 2 to 90 plates, from 3 to 80 plates, from 4 to 70 plates, from 5 to 60 plates, from 8 to 50 plates, or from 10 to 40 plates, and more preferably from 15 to 30 plates. A feed position for the liquid feed is, for example, from 10 to 90%, preferably from 20 to 80%, and more preferably from 30 to 70%, and even more preferably from 40 to 60% of a height of the column facing downward from the top of the column. During the distillation in the product distillation column F, a pressure (absolute pressure) at the top of the column is, for example, from 20 kPa or lower, preferably from 0.1 to 10 kPa, more preferably from 0.3 to 8 kPa, and even more preferably from 0.5 to 5 kPa. The reflux ratio in the product distillation column F is, for example, from 0.05 to 500, preferably from 0.1 to 300, more preferably from 0.2 to 200, even more preferably from 0.5 to 100, from 1 to 50, from 2 to 40, or from 3 to 30, and particularly preferably from 4 to 25.

In FIG. 1, in charging the product column F, the column top vapor from the dealkalization column E is condensed in the condenser E-1, and the resulting condensed liquid is fed, but the column top vapor from the dealkalization column E may be directly fed to the product column F.

The concentration of 1,3-butylene glycol in the liquid feed into the product column F is 97% or higher, preferably 98% or higher, and more preferably 99% or higher. The concentration of 1,3-butylene glycol in the liquid feed into the product column F can be improved, for example, by adjusting the distillation conditions of the dehydration column A, providing a dealcoholization column (low boiling substance removal column) before the dehydration column A, and adjusting the distillation conditions thereof, or adjusting the distillation conditions of the high boiling substance removal column C. For example, it is possible to increase the purity of 1,3-butylene glycol in liquid feed into the product column F by increasing the reflux ratio of the dealcoholization column (low boiling substance removal column), the dehydration column A, and/or the high boiling substance removal column C or increasing the number of plates. Note that the concentration of 1,3-butylene glycol is an area proportion (area%) of a peak of 1,3-butylene glycol relative to a total peak area, according to a gas chromatographic analysis performed under conditions set forth below. Conditions for the gas chromatographic analysis are as follows:

Analytical Column: a column with dimethylpolysiloxane as a stationary phase, having a length of 30 m, an inner diameter of 0.25 mm, and a film thickness of 1.0 $\mu$m

Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes

Sample Introduction Temperature: 250°C

Carrier Gas: helium

Column Gas Flow Rate: 1 mL/min

Detector and Detection Temperature: a flame ionization detector (FID), 280°C.

In the manufacturing method according to the present disclosure, the content of acetaldehyde in the liquid feed into the product column F is 500 ppm or lower, and the content of crotonaldehyde therein is 200 ppm or lower. The content of acetaldehyde in the liquid feed into the product column F is preferably 205 ppm or lower (e.g., 200 ppm or lower), more preferably 100 ppm or lower, even more preferably 90 ppm or lower, 80 ppm or lower, 70 ppm or lower, 60 ppm or lower, 50 ppm or lower, 40 ppm or lower, 30 ppm or lower, 20 ppm or lower, or 10 ppm or lower, and particularly preferably 5 ppm or lower, and may be lower than 2 ppm. The content of the crotonaldehyde in the liquid feed into the product column F is preferably 110 ppm or lower, more preferably 100 ppm or lower, even more preferably 80 ppm or lower, 70 ppm or lower, 60 ppm or lower, 50 ppm or lower, 40 ppm or lower, 30 ppm or lower, 20 ppm or lower, 10 ppm or lower, 5 ppm or lower, or 3 ppm or lower, and particularly preferably 2 ppm or lower, and may be lower than 1 ppm. The acetaldehyde content and the crotonaldehyde content in the liquid feed into the product column F can be reduced, for example, by providing a dealcoholization column (low boiling substance removal column) and a dehydration column upstream of the product column F, and adjusting the distillation conditions of the dealcoholization column (low boiling substance removal column) and the dehydration column. For example, increasing the reflux ratio and the number of plates, and the distillation rate in the dealcoholization column (low boiling substance removal column) and the dehydration column can reduce the acetaldehyde content and the crotonaldehyde content of the liquid feed into the product column F. In addition, the acetaldehyde content and the crotonaldehyde content in the liquid feed into the product column F can be reduced by increasing the reaction temperature, increasing the residence time, or increasing the added amount of the base, in the alkaline reaction. Note that the acetaldehyde content and the crotonaldehyde content of the liquid feed into the product column F can be quantified by GC-MS analysis (gas mass spectrometry).

In the manufacturing method according to the present disclosure, the reflux ratio in the product column F [product column reflux amount/product column distilled amount (discharge amount to outside of distillation column)] is higher than 0.1 (e.g., 0.15 or higher). The reflux ratio is preferably 0.2 or higher, more preferably 0.3 or higher, even more preferably 0.4 or higher, 0.5 or higher, 1 or higher, 2 or higher, 3 or higher, 4 or higher, 5 or higher, 6 or higher, 7 or higher, 8 or higher, 9 or higher, 10 or higher, 11 or higher, 12 or higher, 13 or higher, 14 or higher, 15 or higher, 16 or higher, 17 or higher, 18 or higher, 19 or higher, 20 or higher, 25 or higher, 30 or higher, 35 or higher, 40 or higher, or 50 or higher, and particularly preferably 400 or higher (e.g., 500 or higher), from the perspective of increasing the potassium permanganate test value of the 1,3-butylene glycol product.

In the manufacturing method according to the present disclosure, the distillation rate of the product column F is preferably lower than 20 wt.%. Note that the distillation rate refers to a proportion (wt.%) of an amount of liquid extracted from above the feed plate of the product column F (for example, the top of the column) to the outside of the distillation column (when recycled to the previous step which will be described below, including also the amount of liquid recycled) with respect to a charged amount into the product column F.

From the perspective of improving the recovery rate of 1,3-butylene glycol, the distillation rate of the product column F is preferably 15 wt.% or lower, more preferably 12 wt.% or lower, even more preferably 10 wt.% or lower, 8 wt.% or lower, 5 wt.% or lower, 3 wt.% or lower, 2 wt.% or lower, 1 wt.% or lower, 0.8 wt.% or lower, or 0.6 wt.% or lower, and particularly preferably 0.4 wt.% or lower, and may be 0.2 wt.% or lower.

At least a portion of the liquid (hereinafter, sometimes referred to as "distillate") in which the low boiling point component is concentrated, which is extracted from above the feed plate of the product column F, may be recycled to the step prior to the product distillation step (dashed arrow illustrated on the right side of the product column F in FIG. 1). The recovery rate of 1,3-butylene glycol can be improved by recycling at least a portion of the distillate to the step prior to the product distillation step.

Examples of the step prior to the product distillation step include dehydration step and dealcoholization step (low boiling point substance removal step). Note that the distillate from the product column F is not recycled to a high boiling substance removal step in which the distillate from the high boiling substance removal column is purified to obtain a purified product. However, the distillate from the product column F can be recycled to a high boiling substance removal step in which, as side cut of the high boiling substance removal column, a distillate containing a purified product and a low boiling point substance is taken out, and discarded or recycled to an upstream step. The dealcoholization step (low boiling substance removal step) is preferably provided before the dehydration step. Also, when the crude reaction liquid containing 1,3-butylene glycol is a crude reaction liquid obtained by hydrogen reduction of acetaldols, at least a portion of the distillate from the product column may be recycled to the hydrogen reduction step (hydrogenation step) of acetaldols or a step upstream of the hydrogen reduction. At least a portion of the distillate from the product column is recycled to a step of the hydrogen reduction (hydrogenation step) of acetaldols or a step upstream of the hydrogen reduction, and thus a reducing substance such as acetaldehyde or crotonaldehyde contained in the distillate from the product column can be made essentially harmless by hydrogen addition in the hydrogenation. Therefore, it is possible to prevent such reducing substances from accumulating in the purification system and eventually getting mixed in the 1,3-butylene glycol product. This either makes it unnecessary to discharge the distillate from the product column to the outside the system or an amount to be discharged be extremely small, even if the distillate is discharged to the outside of the system. Therefore, it is possible to achieve two effects of improving the product quality and improving the product yield at the same time.

The amount of the distillate recycled to the step prior to the product distillation can be appropriately selected within the range of the amount of distillate. The amount of the distillate recycle to the step prior to the product distillation step is lower than 30 wt.%, for example, with respect to the charged amount into the product column F. Also, from the perspective of improving the 1,3 BG recovery rate in the product column and the yield throughout the process, the amount of the distillate recycled to the step prior to the product distillation step is, for example, 0.01 wt.% or higher, preferably 0.05 wt.% or higher, more preferably 0.1 wt.% or higher, 0.5 wt.% or higher, 1 wt.% or higher, 1.5 wt.% or higher, 2 wt.% or higher, 3 wt.% or higher, 4 wt.% or higher, 5 wt.% or higher, 7 wt.% or higher, or 10 wt.% or higher, and particularly preferably 20 wt.% or higher with respect to the charged amount into the product column F. In view of an increase in equipment due to the increased load onto the recycling destination column, there should be an upper limit on the amount of the distillate to be recycled. The upper limit is preferably 90 wt.% or lower, more preferably 80 wt.% or lower, and even more preferably 70 wt.% or lower, 60 wt.% or lower, 50 wt.% or lower, or 40 wt.% or lower, and may be 30 wt.% or lower, with respect to a charged amount into the product column F.

Thus, according to the manufacturing method of the present disclosure, 1,3-butylene glycol having a very low content of acetaldehyde and crotonaldehyde and a high potassium permanganate test value can be generally manufactured with a high recovery rate. Note that, in the present specification, the recovery rate of 1,3 BG in the product column F is a value (%) determined by the following formula.

$$\{1 - [\text{GC area\% of 1,3 BG in distillate}] \times (\text{distilled amount (part) - amount (part) of distillate recycled}]/(\text{GC area\% of 1,3 BG in liquid feed} \times \text{charged amount (part)}\} \times 100$$

Note that the low boiling point substance and the high boiling point substance may be hydrolyzed by water to produce 1,3 BG, while the high boiling point substance may be produced by polymerization of 1,3 BG. Further, trace impurities may be produced or disappear. Thus, the mass balance in the product column may not always be made. This applies to the dealcoholization column (low boiling substance removal column), the dehydration column, the high boiling substance removal column, and other distillation columns.

1,3-Butylene glycol product

The 1,3-butylene glycol product of the present disclosure can be obtained by the manufacturing method of the present disclosure. The 1,3-butylene glycol product of the present disclosure has a high potassium permanganate test value (PMT) of higher than 10 minutes. The potassium permanganate test value (PMT) is preferably 15 minutes or longer, more preferably 30 minutes or longer, even more preferably 40 minutes or longer, and particularly preferably 50 minutes or longer (particularly, 60 minutes or longer).

Additionally, in the 1,3-butylene glycol product of the present disclosure, the peak area ratio of 1,3-butylene glycol is preferably higher than 98.5%, according to a gas chromatographic analysis (GC analysis) performed under conditions set forth below. Conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

The peak area ratio of 1,3-butylene glycol is preferably 98.6% or higher, more preferably 98.7% or higher, even higher preferably 98.8% or higher, 98.9% or higher, 99% or higher, or 99.1% or higher, and particularly preferably 99.3% or higher (for example, 99.5% or higher), and may be 99.8% or higher.

In the present disclosure, the "(peak) area ratio" means an area proportion (area%) of a specific peak relative to the sum of the areas of all peaks appearing in the chromatogram. In addition, all peaks mean, for example, all of the peaks appearing in the analysis continued until and discontinued at a relative retention time of 7.8, provided that the relative retention time of 1,3-butylene glycol is 1.0.

In addition, in the 1,3-butylene glycol product of the present disclosure, the content of acetaldehyde is preferably 2 ppm or lower. Furthermore, the content of the crotonaldehyde is preferably 1.2 ppm or lower. The acetaldehyde content and the crotonaldehyde content of the 1,3-butylene glycol product can be quantified by GC-MS analysis (gas mass analysis), for example GC-MS analysis under the following conditions. In GC-MS analysis, even very small peaks are all subjected to mass spectrometry, and each component is quantified. Since the analysis is performed for a specific mass, a substance different in mass is not detected even when another impurity overlaps the peak. Therefore, the analysis is more sensitive than GC analysis which will be described below. In the present specification, the unit "ppm" of the content of each component by GC-MS analysis [content of each compound or the like represented by Formula (A) or (B)] means "ppm by weight".

Conditions for GC-MS analysis

Analytical Column: a column with dimethylpolysiloxane as a stationary phase, having a length of 30 m, an inner diameter of 0.25 mm, and a film thickness of 1.0 μm
Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes Sample Introduction Temperature: 250°C
Carrier Gas: helium
Column Gas Flow Rate: 1 mL/min
Ion source temperature: EI 230°C, CI 250°C
Q Pole temperature: 150°C
Sample: subjected to analysis as it was

When the relative retention time of the peak of 1,3-butylene glycol is 1.0 in the GC-MS analysis conditions, a relative retention time of a peak of acetaldehyde is from 0.3 to 0.5, and a relative retention time of a peak of crotonaldehyde is from 0.3 to 0.5.

Furthermore, the acetaldehyde content of the 1,3-butylene glycol product is more preferably 1.7 ppm or lower, even more preferably 1.5 ppm or lower, and particularly preferably 1.2 ppm or lower, 1.0 ppm or lower, 0.7 ppm or lower, 0.5 ppm or lower, or 0.3 ppm or lower (for example, 0.2 ppm or lower). Furthermore, the crotonaldehyde content of the 1,3-butylene glycol product is more preferably 1.0 ppm or lower, even more preferably 0.7 ppm or lower, and particularly preferably 0.5 ppm or lower, 0.3 ppm or lower, or 0.2 ppm or lower (for example, 0.1 ppm or lower).

With the peak area ratio of 1,3-butylene glycol, the acetaldehyde content, and the crotonaldehyde content in the 1,3-butylene glycol product being within the above ranges, a high-quality and high-quality 1,3-butylene glycol product is provided.

Moisturizer and cosmetic product

A moisturizer of the present disclosure contains the 1,3-butylene glycol product described above. Therefore, the moisturizer has excellent moisturizing performance. The moisturizer of the present disclosure may contain a component other than the 1,3-butylene glycol product described above, such as a moisturizer component other than the 1,3-butylene glycol product described above. In the moisturizer of the present disclosure, the content of the 1,3-butylene glycol product described above is, for example, 10 wt.% or higher, preferably 30 wt.% or higher, more preferably 50 wt.% or higher, even more preferably 80 wt.% or higher, and particularly preferably 90 wt.% or higher, and the moisturizer may contain only the 1,3-butylene glycol product described above.

A cosmetic of the present disclosure contains the moisturizer described above. The blending amount of the 1,3-butylene glycol product in the cosmetic product of the present disclosure is any amount in which the moisturizing performance can be exhibited according to the type and form of cosmetic. The blending amount of the 1,3-butylene glycol product in the cosmetic product of the present disclosure is, for example, from 0.01 to 40 wt.%, preferably from 0.1 to 30 wt.%, more preferably from 0.2 to 20 wt.%, even more preferably from 0.5 to 15 wt.%, and particularly preferably from 1 to 10 wt.%.

The cosmetic product of the present disclosure may contain, in addition to the 1,3-butylene glycol product, for example, another moisturizer; an oil, such as a vegetable oil, a hydrocarbon oil, a higher fatty acid, a higher alcohol, or a silicone; a surfactant, such as an anionic surfactant, a cationic surfactant, an amphoteric surfactant, or a nonionic surfactant; a preservative, a sequestrant, a thickener, a powder, an ultraviolet absorber, an ultraviolet blocker, a fragrance, or a pH adjuster; or a medicinal ingredient or bioactive component, such as a vitamin preparation, a skin activator, a blood circulation promoter, a skin-lightening preparation, an antibacterial agent, or an antiinflammatory agent.

The cosmetic product of the present disclosure can be a skin cosmetic product, such as a lotion, an emulsion, a cream, a gel, a pack, or a mask; or a hair cosmetic product, such as a shampoo, a rinse, or a hair restorer. In addition, the cosmetic product may be a sunscreen cosmetic product, a make-up cosmetic product or the like. Furthermore, the cosmetic product can be a pharmaceutical product or quasi drug containing a medical component.

The cosmetic product of the present disclosure can be manufactured by utilizing a method known per se.

Each aspect disclosed in the present specification can be combined with any other feature disclosed herein. Note that each of the configurations, combinations thereof, and the like in each of the embodiments are an example, and various additions, omissions, and other changes may be made as appropriate without departing from the spirit of the present disclosure. The present disclosure is not limited by the embodiments and is limited only by the claims.

Examples

Hereinafter, the present disclosure will be described more specifically with reference to examples, but the present disclosure is not limited by these examples. "Parts" used in the examples means "parts by weight" unless otherwise specified. Gas chromatographic analysis (GC analysis), GC-MS analysis (gas mass spectrometry), and potassium permanganate test were performed by the method which will be described below.

Example 1

The method of manufacturing 1,3-butylene glycol will be described using FIG. 1.

Relative to 100 parts of an acetaldol solution containing 30 wt.% of water (mixed solution of 69 parts of acetaldol and 29 parts of water, containing a total of 2 parts of low boiling and high boiling impurities, Na salt: lower than 0.1 parts) as a raw material, 10 parts of hydrogen were charged into a reactor for liquid-phase hydrogen reduction, and 15 parts of Raney nickel were added as a catalyst. The reactor was kept at 120°C and 10 MPa (gauge pressure), and liquid-phase hydrogen reduction was performed. After the catalyst was separated, the liquid after the reaction was neutralized with sodium hydroxide, and crude 1,3-butylene glycol (1) containing low boiling impurities and water was obtained.

Note that the acetaldol solution containing 30 wt.% of water used as the raw material was manufactured by stirring acetaldehyde and water in the presence of 100 ppm by weight NaOH at 30°C at a residence time of 10 hours and dimerizing the acetaldehyde [acetaldehyde polymerization (aldol condensation of acetaldehyde)].

The crude 1,3-butylene glycol (1) (corresponding to "X-1" in FIG. 1) was charged into the dehydration column A. In the dehydration column A, water was extracted from the top of the column, and 15 parts of fresh water was added as reflux water relative to 100 parts of the liquid feed amount. The pressure at the top of the column was adjusted to 7 kPa (absolute pressure), and crude 1,3-butylene glycol (2) was obtained from the bottom of the column, the crude 1,3-butylene glycol (2) containing 1 wt.% of water and having a total area ratio of impurity peaks with shorter retention times (RT) than 1,3-butylene glycol of 1.8% in the GC analysis described later. The water extracted from the top of the column was discharged (corresponding to "X-2" in FIG. 1).

The crude 1,3-butylene glycol (2) was then charged into the desalting column B. In the desalting column B, a salt, a high boiling point substance, and a portion of 1,3-butylene glycol were discharged as the evaporation residue from the bottom of the column (corresponding to "X-3" in FIG. 1). The discharge amount of the evaporation residue was 5 parts relative to 100 parts of the liquid feed amount. Meanwhile, from the top of the column was obtained crude 1,3-butylene glycol (3) containing 1,3-butylene glycol, a low boiling point substance, and a portion of a high boiling point substance.

The crude 1,3-butylene glycol (3) was then charged into the high boiling substance removal column C. In the high boiling substance removal column C, a high boiling point substance and a portion of 1,3-butylene glycol were discharged from the bottom of the column (corresponding to "X-4" in FIG. 1). The discharge amount from the bottom of the column was 20 parts relative to 100 parts of the liquid feed amount. Meanwhile, 80 parts of crude 1,3-butylene glycol (4) containing a low boiling point substance was obtained, as a distillate, from the top of the column.

The crude 1,3-butylene glycol (4) was then charged into the alkaline reactor D. At this time, a 20 wt.% sodium hydroxide aqueous solution was added to give a concentration of sodium hydroxide of 0.1 wt.% relative to the liquid feed. The reaction temperature was maintained at 120°C in the alkaline reactor D, and a reaction was performed at a residence time of 20 minutes.

A crude reaction liquid exiting the alkaline reactor D was then charged into the dealkalization column E. In the dealkalization column E, sodium hydroxide, a high boiling point substance, and a portion of 1,3-butylene glycol were discharged from the bottom of the column (corresponding to "X-5" in FIG. 1). The discharge amount from the bottom of the column was 10 parts relative to 100 parts of the liquid feed amount. Meanwhile, from the top of the column were obtained 90 parts of crude 1,3-butylene glycol (5) containing 1,3-butylene glycol and a low boiling point substance. The crude 1,3-butylene glycol (5) containing 1,3-butylene glycol and a low boiling point substance was subjected to GC analysis and GC-MS analysis. As a result, the GC area ratio of 1,3-butylene glycol was 97%, the content of acetaldehyde was 43 ppm, and the content of crotonaldehyde was 15 ppm.

The crude 1,3-butylene glycol (5) was then charged into the product distillation column F. In the product distillation column F, 10 parts of the low boiling point substance and a portion of 1,3-butylene glycol relative to 100 parts of the liquid feed amount were distilled off from the top of the column (corresponding to "X-6" in FIG. 1), and the entire amount was discharged to the outside of the system. The operation was performed at a reflux ratio (reflux amount/distilled amount) of 0.3 at that time, and 90 parts (distillation rate: 10 wt.%) of a 1,3-butylene glycol product was obtained from the bottom of the column (corresponding to "Y" in FIG. 1).

The obtained 1,3-butylene glycol product was subjected to GC analysis and GC-MS analysis. As a result, the GC area ratio of 1,3-butylene glycol was 98.6%, the content of acetaldehyde (AD) was 2 ppm, and the content of crotonaldehyde (CR) was 1.2 ppm. The potassium permanganate test value was 30 minutes. The 1,3-butylene glycol recovery rate in the product column F was 92%.

Example 2

Ninety (90) parts of a 1,3-butylene glycol product was obtained from the bottom of the product column F by the same method as in Example 1 except that the reflux ratio of the product column F was changed to 1. The obtained 1,3-butylene glycol product was subjected to GC analysis and GC-MS analysis. As a result, the GC area ratio of 1,3-butylene glycol was 98.7%, the content of acetaldehyde was 1.5 ppm, and the content of crotonaldehyde was 0.9 ppm. The potassium permanganate test value was 35 minutes. The 1,3-butylene glycol recovery rate in the product column F was 92%.

Example 3

Ninety (90) parts of a 1,3-butylene glycol product (distillation rate: 10 wt.%) was obtained from the bottom of the product column F by the same method as in Example 1 except that the reflux ratio of the dehydration column A was 1, and that the distillate from the product column F was recycled, in the entire amount, to the reactor for hydrogen reduction in place of extraction of the distillate from the product column F to the outside of the system. The obtained 1,3-butylene glycol product was subjected to GC analysis and GC-MS analysis. As a result, the GC area ratio of 1,3-butylene glycol was 98.7%, the content of acetaldehyde was 1.5 ppm, and the content of crotonaldehyde was 0.9 ppm. The potassium permanganate test value was 35 minutes. The 1,3-butylene glycol recovery rate in the product column F was 99% or higher.

Example 4

Ninety (90) parts of a 1,3-butylene glycol product was obtained from the bottom of the product column F by the same method as in Example 3 except that the reflux ratio of the product column F was 10. The obtained 1,3-butylene glycol product was subjected to GC analysis and GC-MS analysis. As a result, the GC area ratio of 1,3-butylene glycol was 98.8%, the content of acetaldehyde was 0.8 ppm, and the content of crotonaldehyde was 0.7 ppm. The potassium permanganate test value was 40 minutes. The 1,3-butylene glycol recovery rate in the product column F was 99% or higher.

Example 5

Ninety (90) parts of a 1,3-butylene glycol product was obtained from the bottom of the product column F by the same method as in Example 3 except that the reflux ratio of the product column F was 20. The obtained 1,3-butylene glycol product was subjected to GC analysis and GC-MS analysis. As a result, the GC area ratio of 1,3-butylene glycol was 98.8%, the content of acetaldehyde was 0.5 ppm, and the content of crotonaldehyde was 0.5 ppm. The potassium permanganate test value was 45 minutes. The 1,3-butylene glycol recovery rate in the product column F was 99% or higher.

Example 6

Ninety (90) parts of a 1,3-butylene glycol product was obtained from the bottom of the product column F by the same method as in Example 3 except that the reflux ratio of the product column F was 50. The obtained 1,3-butylene glycol product was subjected to GC analysis and GC-MS analysis. As a result, the GC area ratio of 1,3-butylene glycol was 98.8%, the content of acetaldehyde was 0.2 ppm, and the content of crotonaldehyde was 0.2 ppm. The potassium permanganate test value was 55 minutes. The 1,3-butylene glycol recovery rate in the product column F was 99% or higher.

Example 7

The same operation as in Example 3 was performed except that a liquid having a peak area ratio of 1,3-butylene glycol of 98%, an acetaldehyde content of 53 ppm and a crotonaldehyde content of 41 ppm was used as the liquid feed into the product column F, that the reflux ratio of the product column F was 1, that the distilled amount from the top of the column was 1 part, and that 99 parts of a 1,3-butylene glycol product was obtained from the bottom of the product column F (distillation rate: 1 wt.%) (the distillate was recycled, in the entire amount, to the reactor for hydrogen reduction). The obtained 1,3-butylene glycol product was subjected to GC analysis and GC-MS analysis. As a result, the GC area ratio of 1,3-butylene glycol was 98.6%, the content of acetaldehyde was 1 ppm, and the content of crotonaldehyde was 1 ppm. The potassium permanganate test value was 40 minutes. The 1,3-butylene glycol recovery rate in the product column F was 99% or higher.

Example 8

The same operation as in Example 7 was performed except that the reflux ratio in the product column F was 5. The obtained 1,3-butylene glycol product was subjected to GC analysis and GC-MS analysis. As a result, the GC area ratio of 1,3-butylene glycol was 98.7%, the acetaldehyde content was 0.9 ppm, and the crotonaldehyde content was 0.8 ppm. The potassium permanganate test value was 45 minutes. The 1,3-butylene glycol recovery rate in the product column F was 99% or higher.

Example 9

The same operation as in Example 7 was performed except that the reflux ratio in the product column F was 10. The obtained 1,3-butylene glycol product was subjected to GC analysis and GC-MS analysis. As a result, the GC area ratio of 1,3-butylene glycol was 98.7%, the acetaldehyde content was 0.5 ppm, and the crotonaldehyde content was 0.3 ppm. The potassium permanganate test value was 50 minutes. The 1,3-butylene glycol recovery rate in the product column F was 99% or higher.

Example 10

The same operation as in Example 7 was performed except that the reflux ratio in the product column F was 100. The obtained 1,3-butylene glycol product was subjected to GC analysis and GC-MS analysis. As a result, the GC area ratio of 1,3-butylene glycol was 98.7%, the acetaldehyde content was lower than 0.2 ppm, and the crotonaldehyde content was lower than 0.1 ppm. The potassium permanganate test value was 60 minutes. The 1,3-butylene glycol recovery rate in the product column F was 99% or higher.

Example 11

The same operation as in Example 7 was performed except that the reflux ratio in the product column F was 500. The obtained 1,3-butylene glycol product was subjected to GC analysis and GC-MS analysis. As a result, the GC area ratio of 1,3-butylene glycol was 98.7%, the acetaldehyde content was lower than 0.2 ppm, and the crotonaldehyde content was lower than 0.1 ppm. The potassium permanganate test value was 65 minutes. The 1,3-butylene glycol recovery rate in the product column F was 99% or higher.

Example 12

The same operation as in Example 3 was performed except that a liquid having a peak area ratio of 1,3-butylene glycol of 99%, an acetaldehyde content of 5 ppm and a crotonaldehyde content of 2 ppm was used as the liquid feed into the product column F, that the reflux ratio of the product column F was 10, that the distilled amount from the top of the column was 0.3 parts, and that 99.7 parts of a 1,3-butylene glycol product was obtained from the bottom of the product column F (distillation rate: 0.3 wt.%) (the distillate was recycled, in the entire amount, to the reactor for hydrogen reduction). The obtained 1,3-butylene glycol product was subjected to GC analysis and GC-MS analysis. As a result, the GC area ratio of 1,3-butylene glycol was 99.2%, the acetaldehyde content was 0.3 ppm, and the crotonaldehyde content was 0.2 ppm. The potassium permanganate test value was 55 minutes. The 1,3-butylene glycol recovery rate in the product column F was 99% or higher.

Example 13

The same operation as in Example 12 was performed except that the distilled amount from the top of the column was 5 parts, and that 95 parts of a 1,3-butylene glycol product was obtained from the bottom of the product column F (distillation rate: 5 wt.%) (the distillate was recycled, in the entire amount, to the reactor for hydrogen reduction). The obtained 1,3-butylene glycol product was subjected to GC analysis and GC-MS analysis. As a result, the GC area ratio of 1,3-butylene glycol was 99.5%, the acetaldehyde content was lower than 0.2 ppm, and the crotonaldehyde content was lower than 0.1 ppm. The potassium permanganate test value was 60 minutes. The 1,3-butylene glycol recovery rate in the product column F was 99% or higher.

Example 14

The same operation as in Example 3 was performed except that the distillate from the product column F was recycled to the acetaldol manufacture in place of the reactor for hydrogen reduction As a result, the quality of the obtained 1,3-butylene glycol product and the 1,3-butylene glycol recovery rate in the product column F were all identical to those in Example 3.

Example 15

This is an example of a relatively high content of acetaldehyde and crotonaldehyde in the liquid feed into the product column (when the charged amount of hydrogen to the hydrogen addition is small, and the reflux ratio in the dealcoholization step is small). The reflux ratio of the product column F was 10, the distilled amount from the top of the column was 10 parts, and 90 parts of a 1,3-butylene glycol product was obtained from the bottom of the product column F (distillation rate: 10 wt.%). The obtained 1,3-butylene glycol product was subjected to GC analysis and GC-MS analysis. As a result, the GC area ratio of 1,3-butylene glycol was 99.4%, the acetaldehyde content was 1.2 ppm, and the crotonaldehyde content was 1.1 ppm. The potassium permanganate test value was 35 minutes. The 1,3-butylene glycol recovery rate in the product column F was 99% or higher.

14

Comparative Example 1

The same operation as in Example 1 was performed except that the reflux ratio of the product column F was 0.05, and 80 parts of a 1,3-butylene glycol product was obtained from the bottom of the product column F (distillation rate: 20 wt.%). The obtained 1,3-butylene glycol product was subjected to GC analysis and GC-MS analysis. As a result, the GC area ratio of 1,3-butylene glycol was 98.5%, the content of acetaldehyde was 6 ppm, and the content of crotonaldehyde was 5 ppm. The potassium permanganate test value was 0 minutes. The 1,3-butylene glycol recovery rate in the product column F was 82%.

Comparative Example 2

The same operation as in Example 1 was performed except that the reflux ratio of the product column F was 0.1, and 80 parts of a 1,3-butylene glycol product was obtained from the bottom of the product column F (distillation rate: 20 wt.%). The obtained 1,3-butylene glycol product was subjected to GC analysis and GC-MS analysis. As a result, the GC area ratio of 1,3-butylene glycol was 98.6%, the content of acetaldehyde was 4 ppm, and the content of crotonaldehyde was 3 ppm. The potassium permanganate test value was 5 minutes. The 1,3-butylene glycol recovery rate in the product column F was 82%.

Reference Example 1

The same operation as in Example 1 was performed except that the reflux ratio of the product column F was 0.1, and 70 parts of a 1,3-butylene glycol product was obtained from the bottom of the product column F (distillation rate: 30 wt.%). The obtained 1,3-butylene glycol product was subjected to GC analysis and GC-MS analysis. As a result, the GC area ratio of 1,3-butylene glycol was 98.7%, the acetaldehyde content was 2 ppm, and the crotonaldehyde content was 1.2 ppm. The potassium permanganate test value was 30 minutes. The 1,3-butylene glycol recovery rate in the product column F was 72%.

Gas chromatographic analysis

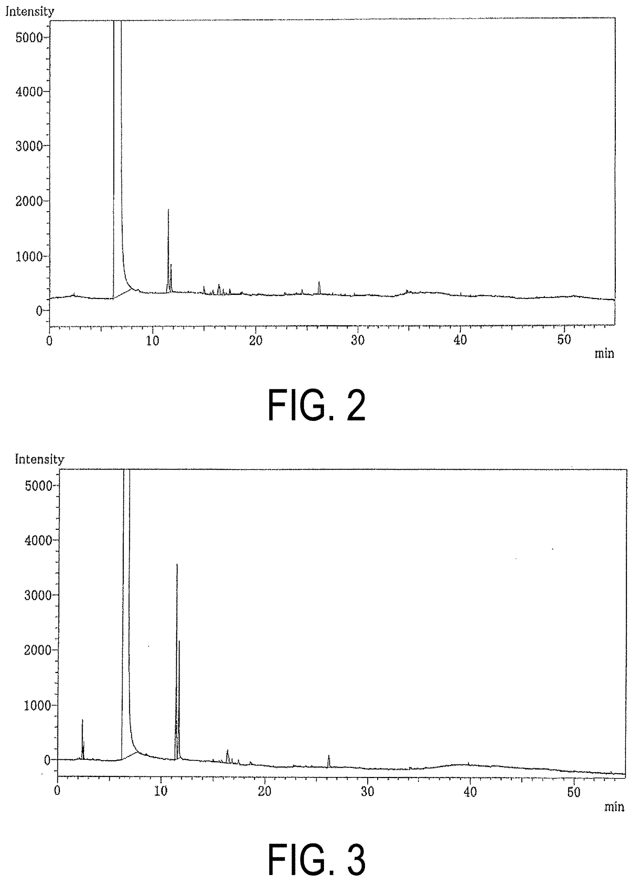

A gas chromatographic analysis of the target 1,3-butylene glycol product was performed under the conditions below. A chromatogram of the gas chromatographic analysis of the 1,3-butylene glycol product in Example 13 is shown in FIG. 2. In addition, a chromatogram of the gas chromatographic analysis of the 1,3-butylene glycol product in Comparative Example 2 is shown in FIG. 3.

Conditions for the gas chromatographic analysis are as follows:

Analytical Instrument: Shimadzu GC 2010
Analytical Column: column with dimethylpolysiloxane as a stationary phase (a length of 30 m, an inner diameter of 0.25 mm, and a film thickness of 1.0 $\mu$m) (Agilent J&W GC column - DB-1, available from Agilent Technologies Japan, Ltd.)
Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes Sample Introduction and Temperature: split sample introduction, 250°C Gas Flow Rate of Split and Carrier Gas: 23 mL/min, helium
Column Gas Flow Rate and Carrier Gas: 1 mL/min, helium
Detector and Temperature: a flame ionization detector (FID), 280°C.
Injection Sample: 0.2 $\mu$L of a 80 wt.% 1,3-butylene glycol product aqueous solution

GC-MS analysis

Analytical Instrument: Agilent 6890A-GC/5973A-MSD
Analytical Column: a column with dimethylpolysiloxane as a stationary phase, having a length of 30 m, an inner diameter of 0.25 mm, and a film thickness of 1.0 $\mu$m
Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes Sample Introduction Temperature: 250°C
Carrier Gas: helium
Column Gas Flow Rate: 1 mL/min
Ion source temperature: EI 230°C, CI 250°C
Q Pole temperature: 150°C
Sample: subjected to analysis as it was

Potassium permanganate test

In the present specification, the potassium permanganate test value (PMT) is a value measured in accordance with the visual colorimetric procedure of JIS K1351 (1993).

Considerations of results

Results of the above comparative examples and examples are shown in Table 1 and Table 2.

[Table 1]

Table 1

| | | Comparative Example 1 | Comparative Example 2 | Reference Example 1 | Example 1 | Example 2 | Example 3 | Example 4 | Example 5 | Example 6 |
|---|---|---|---|---|---|---|---|---|---|---|
| Charge into product column F | Part | 100 | ← | ← | ← | ← | ← | ← | ← | ← |
| | 1,3 BG GC area% | 97 | ← | ← | ← | ← | ← | ← | ← | ← |
| | AD ppm | 43 | ← | ← | ← | ← | ← | ← | ← | ← |
| | CR ppm | 15 | ← | ← | ← | ← | ← | ← | ← | ← |
| Reflux ratio of product column F | | 0.05 | 0.1 | 0.1 | 0.3 | 1 | 1 | 10 | 20 | 50 |
| Distillation from product column F | Part | 20 | 20 | 30 | 10 | 10 | 10 | 10 | 10 | 10 |
| | Recycle | Absent | Absent | Absent | Absent | Absent | Present | Present | Present | Present |
| Bottom (product) from product column F | Part | 80 | 80 | 70 | 90 | 90 | 90 | 90 | 90 | 90 |
| | 1,3 BG GC area% | 98.5 | 98.6 | 98.7 | 98.6 | 98.7 | 98.7 | 98.8 | 98.8 | 98.8 |
| | AD ppm | 6 | 4 | 2 | 2 | 1.5 | 1.5 | 0.8 | 0.5 | 0.2 |
| | CR ppm | 5 | 3 | 1.2 | 1.2 | 0.9 | 0.9 | 0.7 | 0.5 | 0.2 |
| | PMT min | 0 | 5 | 30 | 30 | 35 | 35 | 40 | 45 | 55 |
| | 1,3 BG recovery rate % | 82 | 82 | 72 | 92 | 92 | 99 or higher | 99 or higher | 99 or higher | 99 or higher |

[Table 2]

Table 2

| | | Example 7 | Example 8 | Example 9 | Example 10 | Example 11 | Example 12 | Example 13 | Example 15 |
|---|---|---|---|---|---|---|---|---|---|
| | Part | 100 | ← | ← | ← | ← | 100 | ← | 100 |
| Charge into product column F | 1,3 BG GC area% | 98 | ← | ← | ← | ← | 99 | ← | 99 |
| | AD ppm | 53 | ← | ← | ← | ← | 5 | ← | 205 |
| | CR ppm | 41 | ← | ← | ← | ← | 2 | ← | 110 |
| Reflux ratio of product column F | | 1 | 5 | 10 | 100 | 500 | 10 | 10 | 10 |
| Distillation from product column F | Part | 1 | 1 | 1 | 1 | 1 | 0.3 | 5 | 10 |
| | Recycle | Present | Present | Present | Present | Present | Present | Present | Present |
| Bottom (product) from product column F | Part | 99 | 99 | 99 | 99 | 99 | 99.7 | 95 | 90 |
| | 1,3 BG GC area% | 98.6 | 98.7 | 98.7 | 98.7 | 98.7 | 99.2 | 99.5 | 99.4 |
| | AD ppm | 1 | 0.9 | 0.5 | lower than 0.2 | lower than 0.2 | 0.3 | lower than 0.2 | 1.2 |
| | CR ppm | 1 | 0.8 | 0.3 | lower than 0.1 | lower than 0.1 | 0.2 | lower than 0.1 | 1.1 |
| | PMT min | 40 | 45 | 50 | 60 | 65 | 55 | 60 | 35 |
| | 1,3 BG recovery rate % | 99 or higher | 99 or higher | 99 or higher | 99 or higher | 99 or higher | 99 or higher | 99 or higher | 99 or higher |

19

EP 4 083 004 A1

From Comparative Examples 1 and 2, at the distillation rate of 20 wt.%, the effect of removing acetaldehyde (AD) and crotonaldehyde (CR), and the potassium permanganate test value (PMT) of the product is not satisfactory even when the reflux ratio is 0.1. When the distillation rate is 30 wt.% (Reference Example 1), at the reflux ratio of 0.1, the product satisfies a PMT of 30 minutes, but the 1,3 BG recovery rate in the product column is very deteriorated, i.e., 72%, which is economically disadvantageous.

From Comparative Examples 1 and 2, Reference Example 1, and Example 1, when the reflux ratio of the product column is changed from 0.1 to 0.3, the amounts of AD and CR removed from the product column distillate are increased at a distillation rate of 10 wt.% (recovery rate: 92%). Therefore, the concentrations of AD and CR in the product are reduced, and, as result, the PMT of the bottom (product) from the product column is improved.

It is clear that, even at a relatively low reflux ratio, the quality can be maintained with a certain degree of recovery rate, when the liquid feed composition (in particular, AD and CR), the reflux ratio, and the distilled amount are controlled.

From Examples 1 and 2, when the reflux ratio of the product column is changed from 0.3 to 1, the amounts of AD and CR removed from the product column distillate are further increased. Therefore, the concentrations of AD and CR in the product are reduced, and, as result, the PMT of the bottom (product) from the product column is further improved.

In comparison of Examples 2, 3 and 14, the product column distillate was recycled, and thus almost equivalent product quality was obtained when the 1,3 BG recovery rate was improved. This is because the AD and CR contained in the product column distillate were hydrogenated in the reactor for hydrogen reduction, in Example 3. This is also because, in Example 14, the AD and CR contained in the product column distillate were dimerized in the acetaldol manufacture.

From Examples 3 to 6, when the reflux ratio of the product column is increased, the concentrations of AD and CR in the product are further reduced, and the PMT quality is improved.

From Examples 7 to 11, when the purity of the liquid feed into the product column is increased and the concentrations of AD and CR are decreased, the product quality is improved even when the reflux ratio of the product column is decreased and the distilled amount is decreased. Furthermore, from Examples 7 to 11, it is clear that, when the reflux ratio is increased, the quality of the product is improved, but that, when the reflux ratio is increased to 500, the improvement in product quality becomes gradual, and the effect is reduced by the amount of energy increase due to the increase in reflux amount.

It is clear, from Examples 4, 9, and 12 that the product quality can be maintained even when the distilled amount and the purity in the liquid feed are changed under a constant reflux amount. It is clear that the reduction in amount of the distillate recycled is limited, though depending on the concentrations of AD and CR in the liquid feed, because the amounts of AD and CR removed are extremely reduced if the amount of the distillate recycled is too low, but that the quality can be maintained as long as the amount is approximately 0.1 wt.%. As the amount of the distillate recycled is lower, the amount of increase in facility size due to increase in treated amount due to the recycle to the previous step is reduced, and thus the amount of the distillate recycled is preferably as low as possible. However, it is desirable to determine an optimum value, taking into consideration the entire balance, the quality, and the like.

Example 15 shows that, even when the content of AD and CR in the liquid feed into the product column is high to a certain degree, the product quality is maintained when the product column reflux amount and distilled amount are relatively large.

Note that, among 1,3-butylene glycol products obtained by known methods, there was no product having a high potassium permanganate test value (PMT) as in the present disclosure.

As a summary of the above, configurations and variations of the present disclosure are described below.

[1] A method for manufacturing 1,3-butylene glycol, which is a method for obtaining purified 1,3-butylene glycol from a crude reaction liquid containing 1,3-butylene glycol, the method including:

a dehydration step of removing water by distillation; a high boiling substance removal step of removing a high boiling point component by distillation; and a product distillation step of obtaining purified 1,3-butylene glycol, wherein, in the product distillation step, a product column is used in which a liquid feed having a 1,3-butylene glycol concentration of 97% or higher (or 98% or higher, or 99% or higher), an acetaldehyde content of 500 ppm or lower (or 200 ppm or lower, 100 ppm or lower, 90 ppm or lower, 80 ppm or lower, 70 ppm or lower, 60 ppm or lower, 50 ppm or lower, 40 ppm or lower, 30 ppm or lower, 20 ppm or lower, 10 ppm or lower, 5 ppm or lower, or lower than 2 ppm), and a crotonaldehyde content of 200 ppm or lower (or 110 ppm or lower, 100 ppm or lower, 80 ppm or lower, 70 ppm or lower, 60 ppm or lower, 50 ppm or lower, 40 ppm or lower, 30 ppm or lower, 20 ppm or lower, 10 ppm or lower, 5 ppm or lower, 3 ppm or lower, 2 ppm or lower, or lower than 1 ppm) is distilled under a condition of a reflux ratio of higher than 0.1 (or 0.15 or higher), and a liquid concentrated with acetaldehyde and crotonaldehyde is distilled off from above a feed plate, and 1,3-butylene glycol is extracted from below the feed plate.

[2] The method for manufacturing 1,3-butylene glycol according to [1], wherein the crude reaction liquid containing 1,3-butylene glycol is: a crude reaction liquid obtained by hydrogen reduction of an acetaldol (or a crude reaction liquid obtained by hydrolyzation of 1,3-butylene oxide; a crude reaction liquid obtained by selective hydrocracking of erythritol; a crude reaction liquid obtained by selective water addition to butadiene; a crude reaction liquid obtained by hydrogen addition to n-butanal-3-one; a crude reaction liquid obtained by hydrogen addition to 1-butanol-3-one; a crude reaction liquid obtained by hydrogen addition to 3-hydroxy-1-butanoic acid; a crude reaction liquid obtained by hydrogen addition to β-butyrolactone; or a crude reaction liquid obtained by hydrogen addition to diketene.

[3] The method for manufacturing 1,3-butylene glycol according to [2], wherein the acetaldol used as a raw material in reduction (hydrogenation) of the acetaldol is any of acetaldol; its cyclic dimer paraldol; aldoxane as a cyclic trimer of acetaldehyde; and mixtures of these.

[4] The method for manufacturing 1,3-butylene glycol according to [2] or [3], wherein the crude reaction liquid containing an acetaldol is subjected to simple evaporation, distillation, or hydrogen addition to remove aldehydes, followed by a hydrogenation step.

[5] The method for manufacturing 1,3-butylene glycol according to any one of [2] to [4], wherein a content of the acetaldol used as the raw material in reduction (hydrogenation) of the acetaldol is 30 wt.% or higher (e. g., from 30 to 99 wt.%) (or 40 wt.% or higher (e. g., from 40 to 98 wt.%), 50 wt.% or higher (e. g., from 50 to 97 wt.%), 60 wt.% or higher (e. g., from 60 to 95 wt.%), from 65 to 90 wt.%, from 70 to 90 wt.%, or 75 to 90 wt.%).

[6] The method for manufacturing 1,3-butylene glycol according to any one of [2] to [5], wherein the raw material for the reduction (hydrogenation) of the acetaldol contains water, a content of the water is 2 wt.% or higher (or 5 wt.% or higher, 10 wt.% or higher, or 15 wt.% or higher), and an upper limit of the content of the water is 90 wt.% (or 80 wt.%, 70 wt.%, 60 wt.%, 50 wt.%, 40 wt.%, 30 wt.%, or 20 wt.%).

[7] The method for manufacturing 1,3-butylene glycol according to any one of [2] to [6], wherein an amount of a hydrogenation catalyst for the reduction (hydrogenation) of the acetaldol is from 1 to 30 parts by weight (or from 4 to 25 parts by weight, from 8 to 20 parts by weight, or from 12 to 18 parts by weight) relative to 100 parts by weight of the raw material for hydrogenation.

[8] The method for manufacturing 1,3-butylene glycol according to any one of [2] to [7], wherein an amount of hydrogen used in the reduction (hydrogenation) reaction of the acetaldol is from 0.5 to 40 parts by weight (or from 1 to 30 parts by weight, from 4 to 20 parts by weight, or from 8 to 12 parts by weight) relative to 100 parts by weight of the raw material for hydrogenation.

[9] The method for manufacturing 1,3-butylene glycol according to any one of [2] to [8], wherein a pressure (total pressure, gauge pressure) in a reaction system in the reduction (hydrogenation) reaction of the acetaldol is from 9 to 70 MPa (or from 10 to 40 MPa).

[10] The method for manufacturing 1,3-butylene glycol according to any one of [2] to [9], wherein a hydrogen pressure (partial pressure of hydrogen) in the reaction system in the reduction (hydrogenation) reaction of the acetaldol is from 7 to 60 MPa (or 10 to 30 MPa).

[11] The method for manufacturing 1,3-butylene glycol according to any one of [2] to [10], wherein a reaction temperature in the reduction (hydrogenation) reaction of the acetaldol is from 40 to 150°C (or from 50 to 140°C, or from 60 to 130°C).

[12] The method for manufacturing 1,3-butylene glycol according to any one of [2] to [11], wherein a reaction time (residence time) in the reduction (hydrogenation) reaction of the acetaldol is from 10 to 500 minutes (or from 20 to 400 minutes, from 30 to 300 minutes, from 50 to 280 minutes, or from 80 to 250 minutes).

[13] The method for manufacturing 1,3-butylene glycol according to any one of [1] to [12], further including a dealcoholization step of removing a low boiling substance containing alcohols in a process stream containing 1,3-butylene glycol.

[14] The method for manufacturing 1,3-butylene glycol according to any one of [1] to [13], further including a desalting step of removing a salt in a process stream containing 1,3-butylene glycol.

[15] The method for manufacturing 1,3-butylene glycol according to [14], wherein a bottom rate (%) in the a desalting step [(desalting column bottom amount (part)/desalting column charged amount (part)·100] is from 0.1 to 40 wt.% (or from 1 to 35 wt.%, from 2 to 30 wt.%, from 3 to 25 wt.%, from 5 to 20 wt.%, or from 7 to 15 wt.%).

[16] The method for manufacturing 1,3-butylene glycol according to any one of [1] to [15], wherein the high boiling substance removal column used in the high boiling substance removal is a packed column (or perforated-plate column or a bubble column), and a reboiler employed is a natural downward flow thin-film evaporator or a forced-stirring thin-film evaporator.

[17] The method for manufacturing 1,3-butylene glycol according to [16], wherein the number of theoretical plates of the high boiling substance removal column is from 1 to 100 plates (or from 2 to 90 plates, from 3 to 80 plates, from 4 to 70 plates, from 5 to 60 plates, from 8 to 50 plates, from 10 to 40 plates, or from 15 to 30 plates).

[18] The method for manufacturing 1,3-butylene glycol according to any one of [16] or [17], wherein a feed position of the liquid feed is from 10 to 90% (or from 20 to 80%, from 30 to 70%, or from 40 to 60%) of a height of the column facing downward from the top of the high boiling substance removal column.

[19] The method for manufacturing 1,3-butylene glycol according to any one of [16] to [18], wherein, in the distillation in the high boiling substance removal column, a pressure (absolute pressure) at the top of the column is from 0.01 to 50 kPa (or from 0.1 to 30 kPa, or from 0.3 to 20 kPa, or from 0.5 to 10 kPa).

[20] The method for manufacturing 1,3-butylene glycol according to any one of the items [16] to [19], wherein at least a portion of a bottom from the high boiling substance removal column is recycled to a step prior to the high boiling substance removal step.

[21] The method for manufacturing 1,3-butylene glycol according to any one of [16] to [20], wherein a reflux ratio of the high boiling substance removal column is 0.015 or higher (or 0.02 or higher, 0.03 or higher, 0.05 or higher, 0.07 or higher, 0.1 or higher, 0.2 or higher, 0.3 or higher, 0.4 or higher, 0.5 or higher, 0.6 or higher, 0.7 or higher, 0.8 or higher, 0.9 or higher, 1 or higher, 2 or higher, 3 or higher, 4 or higher, 5 or higher, 6 or higher, 7 or higher, 8 or higher, 9 or higher, 10 or higher, 15 or higher, 20 or higher, or 30 or higher).

[22] The method for manufacturing 1,3-butylene glycol according to [21], wherein an upper limit of the reflux ratio is 100 (or 50).

[23] The method for manufacturing 1,3-butylene glycol according to any one of [1] to [22], further including an alkaline treatment step of treating a process stream containing 1,3-butylene glycol with a base.

[24] The method for manufacturing 1,3-butylene glycol according to [23], wherein, in the alkaline treatment, the base is added in an amount of from 0.05 to 10 wt.% (or from 0.1 to 1.0 wt.%) relative to a crude 1,3-butylene glycol stream subjected to the alkaline treatment.

[25] The method for manufacturing 1,3-butylene glycol according to [23] or [24], wherein the base added in the alkaline treatment is an alkali metal compound.

[26] The method for manufacturing 1,3-butylene glycol according to [25], wherein the alkali metal compound is sodium hydroxide (or potassium hydroxide, sodium (bi)carbonate, or potassium (bi)carbonate).

[27] The method for manufacturing 1,3-butylene glycol according to any one of [23] to [26], wherein a reaction temperature of the alkaline treatment is from 90 to 140°C (or from 110 to 130°C), and a reaction residence time is from 5 to 120 minutes (or from 10 to 30 minutes).

[28] The method for manufacturing 1,3-butylene glycol according to any one of [23] to [27], wherein evaporation is performed in an evaporator used in the alkaline treatment under a reduced pressure at the top of the column of 20 kPa or lower (absolute pressure) (or from 0.5 to 10 kPa (absolute pressure)) at from 90 to 120°C.

[29] The method for manufacturing 1,3-butylene glycol according to any one of [1] to [28], wherein the product column is a packed column (or perforated-plate column or a bubble column), and a reboiler employed is a natural downward flow thin-film evaporator or a forced-stirring thin-film evaporator.

[30] The method for manufacturing 1,3-butylene glycol according to any one of [1] to [29], wherein the number of theoretical plates of the product column is from 1 to 100 plates (or from 2 to 90 plates, from 3 to 80 plates, from 4 to 70 plates, from 5 to 60 plates, from 8 to 50 plates, from 10 to 40 plates, or from 15 to 30 plates).

[31] The method for manufacturing 1,3-butylene glycol according to any one of [1] to [30], wherein a feed position of the liquid feed in the product column is from 10 to 90% (or from 20 to 80%, from 30 to 70%, from 40 to 60%) of the height of the column downward from the top of the column, and the pressure (absolute pressure) at the top of the column is from 20 kPa or less (or from 0.1 to 10 kPa, from 0.3 to 8 kPa, or from 0.5 to 5 kPa).

[32] The method for manufacturing 1,3-butylene glycol according to any one of [1] to [31], wherein the reflux ratio in the product column is 0.2 or higher (or 0.3 or higher, 0.4 or higher, 0.5 or higher, 1 or higher, 2 or higher, 3 or higher, 4 or higher, 5 or higher, 6 or higher, 7 or higher, 8 or higher, 9 or higher, 10 or higher, 11 or higher, 12 or higher, 13 or higher, 14 or higher, 15 or higher, 16 or higher, 17 or higher, 18 or higher, 19 or higher, 20 or higher, 25 or higher, 30 or higher, 35 or higher, 40 or higher, 50 or higher, 400 or higher, or 500 or higher).

[33] The method for manufacturing 1,3-butylene glycol according to any one of [1] to [32], wherein the content of acetaldehyde in the liquid feed into the product column is 205 ppm or lower (or 200 ppm or lower, 100 ppm or lower, 90 ppm or lower, 80 ppm or lower, 70 ppm or lower, 60 ppm or lower, 50 ppm or lower, 40 ppm or lower, 30 ppm or lower, 20 ppm or lower, 10 ppm or lower, 5 ppm or lower, or lower than 2 ppm).

[34] The method for manufacturing 1,3-butylene glycol according to any one of [1] to [33], wherein the content of crotonaldehyde in the liquid feed into the product column is 110 ppm or lower (or 100 ppm or lower, 80 ppm or lower, 70 ppm or lower, 60 ppm or lower, 50 ppm or lower, 40 ppm or lower, 30 ppm or lower, 20 ppm or lower, 10 ppm or lower, 5 ppm or lower, 3 ppm or lower, 2 ppm or lower, or lower than 1 ppm).

[35] The method for manufacturing 1,3-butylene glycol according to any one of [1] to [34], wherein a distillation rate in the product column is lower than 20 wt.% (or 15 wt.% or lower, 12 wt.% or lower, 10 wt.% or lower, 8 wt.% or lower, 5 wt.% or lower, 3 wt.% or lower, 2 wt.% or lower, 1 wt.% or lower, 0.8 wt.% or lower, 0.6 wt.% or lower, 0.4 wt.% or lower, or 0.2 wt.% or lower).

[36] The method for manufacturing 1,3-butylene glycol according to any one of [1] to [35], wherein at least a portion of a distillate from the product column is recycled to a step prior to the product distillation step, namely the dehydration step, a dealcoholization step, a low boiling substance removal step, or another step prior to these step.

[37] The method for manufacturing 1,3-butylene glycol according to [36], wherein the crude reaction liquid containing 1,3-butylene glycol is a crude reaction liquid obtained by hydrogen reduction of an acetaldol, and at least a portion of the distillate from the product column is recycled to a step of the hydrogen reduction of an acetaldol or a step upstream of the hydrogen reduction.

[38] The method for manufacturing 1,3-butylene glycol according to[36] or [37], wherein an amount of the distillate from the product column being recycled to a step prior to the product distillation is lower than 30 wt.% (or 90 wt.% or lower, 80 wt.% or lower, 70 wt.% or lower, 60 wt.% or lower, 50 wt.% or lower, 40 wt.% or lower, or 30 wt.% or lower) with respect to a charged amount into the product column within a range not higher than a distilled amount in the product column.

[39] The method for manufacturing 1,3-butylene glycol according to any one of [36] to [38], wherein the amount of the distillate from the product column being recycled to a step prior to the product distillation is 0.01 wt.% or higher (or 0.05 wt.% or higher, 0.1 wt.% or higher, 0.5 wt.% or higher, 1 wt.% or higher, 1.5 wt.% or higher, 2 wt.% or higher, 3 wt.% or higher, 4 wt.% or higher, 5 wt.% or higher, 7 wt.% or higher, 10 wt.% or higher, or 20 wt.% or higher) with respect to the charged amount into the product column within a range not higher than the distilled amount in the product column.

[40] A 1,3-butylene glycol product, having a potassium permanganate test value of longer than 10 minutes (or 5 minutes or longer, 30 minutes or longer, 40 minutes or longer, 50 minutes or longer, or 60 minutes or longer).

[41] The 1,3-butylene glycol product according to [40], having a peak area ratio of 1,3-butylene glycol of higher than 98.5% (or 98.6% or higher, 98.7% or higher, 98.8% or higher, 98.9% or higher, 99% or higher, 99.1% or higher, 99.2% or higher, 99.3%, 99. 5% or higher, or 99.8% or higher), according to a gas chromatographic analysis performed under conditions set forth below,

in which the conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

[42] The 1,3-butylene glycol product according to [40] or [41], wherein a content of acetaldehyde is 2 ppm or lower (or 1.7 ppm or lower, 1.5 ppm or lower, 1.2 ppm or lower, 1.0 ppm or lower, 0.7 ppm or lower, 0.5 ppm or lower, 0.3 ppm or lower, or 0.2 ppm or lower).

[43] The 1,3-butylene glycol product according to any one of [40] to [42], wherein a content of crotonaldehyde is 1.2 ppm or lower (or 1.0 ppm or lower, 0.7 ppm or lower, 0.5 ppm or lower, 0.3 ppm or lower, 0.2 ppm or lower, or 0.1 ppm or lower).

[44] A moisturizer containing the 1,3-butylene glycol product described in any one of [40] to [43].

[45] The moisturizer according to [44], wherein a content of the 1,3-butylene glycol product is 10 wt.% or higher (or 30 wt.% or higher, 50 wt.% or higher, 80 wt.% or higher, 90 wt.% or higher, or 100 wt.% or higher).

[46] A cosmetic product containing the moisturizer described in [44] or [45].

[47] The cosmetic product according to [46], wherein an amount of the 1,3-butylene glycol product blended is from 0.01 to 40 wt.% (or from 0.1 to 30 wt.%, from 0.2 to 20 wt.%, from 0.5 to 15 wt.%, or from 1 to 10 wt.%).

Industrial Applicability

The 1,3-butylene glycol product according to the present disclosure has a high potassium permanganate test value, and is suitably used as a moisturizer and in an application as a raw material for cosmetics. The 1,3-butylene glycol product can be used as a raw material for a moisturizer and a cosmetic product that have excellent moisturizing performance and can maintain high quality for a long period of time.

Reference Signs List

A: Dehydration column

B: Desalting column
C: Distillation column for removing a high boiling point substance (high boiling substance removal column)
D: Alkaline reactor
E: Dealkalization column
F: Product distillation column (product column)
A-1, B-1, C-1, E-1, F-1: Condenser
A-2, C-2, F-2: Reboiler
X-1: Crude 1,3-butylene glycol
X-2: Water (discharged water)
X-3: A salt, a high boiling point substance, and a portion of 1,3-butylene glycol
X-4: A high boiling point substance and a portion of 1,3-butylene glycol
X-5: Sodium hydroxide, a high boiling point substance, and a portion of 1,3-butylene glycol
X-6: A low boiling point substance and a portion of 1,3-butylene glycol
Y: 1,3-butylene glycol product

## Claims

1. A method for manufacturing 1,3-butylene glycol, which is a method for obtaining purified 1,3-butylene glycol from a crude reaction liquid containing 1,3-butylene glycol, the method comprising:

   a dehydration step of removing water by distillation; a high boiling substance removal step of removing a high boiling point component by distillation; and a product distillation step of obtaining purified 1,3-butylene glycol, wherein in the product distillation step, a product column is used in which a liquid feed having a 1,3-butylene glycol concentration of 97% or higher, an acetaldehyde content of 500 ppm or lower, and a crotonaldehyde content of 200 ppm or lower is distilled under a condition of a reflux ratio of higher than 0.1, and a liquid concentrated with acetaldehyde and crotonaldehyde is distilled off from above a feed plate, and 1,3-butylene glycol is extracted from below the feed plate.

2. The method for manufacturing 1,3-butylene glycol according to claim 1, wherein the crude reaction liquid containing 1,3-butylene glycol is a crude reaction liquid obtained by hydrogen reduction of an acetaldol.

3. The method for manufacturing 1,3-butylene glycol according to claim 1 or 2, further comprising an alkaline treatment step of treating a process stream containing 1,3-butylene glycol with a base.

4. The method for manufacturing 1,3-butylene glycol according to any one of claims 1 to 3, further comprising a desalting step of removing a salt in a process stream containing 1,3-butylene glycol.

5. The method for manufacturing 1,3-butylene glycol according to any one of claims 1 to 4, further comprising a dealcoholization step of removing a low boiling substance containing alcohols in a process stream containing 1,3-butylene glycol.

6. The method for manufacturing 1,3-butylene glycol according to any one of claims 1 to 5, wherein the product column has a reflux ratio of 0.2 or higher.

7. The method for manufacturing 1,3-butylene glycol according to any one of claims 1 to 6, wherein the liquid feed into the product column has an acetaldehyde content of 205 ppm or lower.

8. The method for manufacturing 1,3-butylene glycol according to any one of claims 1 to 7, wherein the liquid feed into the product column has a crotonaldehyde content of 110 ppm or lower.

9. The method for manufacturing 1,3-butylene glycol according to any one of claims 1 to 8, wherein the product column has a distillation rate of lower than 20 wt.%.

10. The method for manufacturing 1,3-butylene glycol according to any one of claims 1 to 9, wherein the product column has the number of theoretical plates of from 1 to 100.

11. The method for manufacturing 1,3-butylene glycol according to any one of claims 1 to 10, wherein at least a portion of a distillate from the product column is recycled to a step prior to the product distillation step, namely the dehydration step, a dealcoholization step, a low boiling substance removal step, or another step prior to these steps.

12. The method for manufacturing 1,3-butylene glycol according to clam 11, wherein the crude reaction liquid containing 1,3-butylene glycol is a crude reaction liquid obtained by hydrogen reduction of an acetaldol, and at least a portion of the distillate from the product column is recycled to the hydrogen reduction of an acetaldol or a step upstream of the hydrogen reduction.

13. The method for manufacturing 1,3-butylene glycol according to claim 11 or 12, wherein an amount of the distillate from the product column being recycled to a step prior to the product distillation step is lower than 30 wt.% with respect to a charged amount into the product column within a range not higher than a distilled amount in the product column.

14. The method for manufacturing 1,3-butylene glycol according to any one of claims 11 to 13, wherein an amount of the distillate from the product column being recycled to a step prior to the product distillation step is 0.01 wt.% or higher with respect to a charged amount into the product column within a range not higher than a distilled amount in the product column.

15. A 1,3-butylene glycol product, having a potassium permanganate test value of longer than 10 minutes.

16. The 1,3-butylene glycol product according to claim 15, having, according to a gas chromatographic analysis performed under conditions set forth below, a peak area ratio of 1,3-butylene glycol of higher than 98.5%, wherein the conditions for the gas chromatographic analysis are as follows:

    Analytical Column: a column with dimethylpolysiloxane as a stationary phase, having a length of 30 m, an inner diameter of 0.25 mm, and a film thickness of 1.0 μm
    Heating Conditions: heating from 80°C to 120°C at 5°C/min, then heating again to 160°C at 2°C/min and maintaining for 2 minutes, and further heating to 230°C at 10°C/min and maintaining at 230°C for 18 minutes
    Sample Introduction Temperature: 250°C
    Carrier Gas: helium
    Column Gas Flow Rate: 1 mL/min
    Detector and Detection Temperature: a flame ionization detector (FID), 280°C.

17. The 1,3-butylene glycol product according to claim 15 or 16, having an acetaldehyde content of 2 ppm or lower.

18. The 1,3-butylene glycol product according to any one of claims 15 to 17, having a crotonaldehyde content of 1.2 ppm or lower.

19. A moisturizer comprising the 1,3-butylene glycol product described in any one of claims 15 to 18.

20. A cosmetic product comprising the moisturizer described in claim 19.

FIG. 1

FIG. 2

FIG. 3

## INTERNATIONAL SEARCH REPORT

International application No.

PCT/JP2020/048220

### A. CLASSIFICATION OF SUBJECT MATTER
Int.Cl. C07C29/80(2006.01)i, C07C31/20(2006.01)i, A61Q19/00(2006.01)i,
C07C37/74(2006.01)i, A61K8/34(2006.01)i
FI: A61K8/34, C07C31/20BCSP, A61Q19/00, C07C37/74, C07C29/80
According to International Patent Classification (IPC) or to both national classification and IPC

### B. FIELDS SEARCHED
Minimum documentation searched (classification system followed by classification symbols)
Int.Cl. C07C29/80, C07C31/20, A61Q19/00, C07C37/74, A61K8/34

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2001-213825 A (DAICEL CHEM IND LTD.) 07 August 2001 (2001-08-07), claims, paragraphs [0020]-[0026], fig. 1 | 1-10, 15-20<br>11-14 |
| Y | WO 2000/07969 A1 (DAICEL CHEM IND LTD.) 17 February 2000 (2000-02-17), claims, page 8, lines 19-22 | 11-14 |
| A | JP 2001-213822 A (DAICEL CHEM IND LTD.) 07 August 2001 (2001-08-07), claims | 1-20 |

☐ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 February 2021 | 09 February 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**
Information on patent family members

| International application No. |
| --- |
| PCT/JP2020/048220 |

```
JP 2001-213825 A   07 August 2001     US 2003/0018224 A1
                                      claims, paragraphs [0079]-[0241]
                                      KR 10-2001-0102420 A

WO 2000/07969 A1   17 February 2000   US 6376725 B1
                                      claims, column 5, lines 60-67
                                      EP 1046628 A1
                                      KR 10-0595399 B1

JP 2001-213822 A   07 August 2001     US 2003/0018224 A1
                                      claims
                                      KR 10-2001-0102420 A
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019239974 A
- JP 2019239975 A
- JP 2019239976 A
- JP 2019239977 A
- JP 2019239978 A
- JP 2019239979 A
- JP 2020006660 A
- JP 2020018910 A
- JP 2001213825 A